# EUROPEAN PATENT SPECIFICATION

(11) **EP 3 223 687 B1**
(45) Date of publication and mention of the grant of the patent: **13.03.2019**
(21) Application number: 15823004.5
(22) Date of filing: 24.11.2015
(51) Int. Cl.: A61B 5/00

(54) **DEVICE FOR MEASURING ALLERGIC WHEALS**
VORRICHTUNG ZUR MESSUNG ALLERGISCHER URTICA
DISPOSITIF DE MESURE DE PAPULES ALLERGIQUES

(30) Priority: 24.11.2014 IT MI20142023
(43) Date of publication of application: 04.10.2017
(73) Proprietor: CD Pharma Group S.r.l., 20146 Milano (IT)
(72) Inventor: GIORDANO, Fabio, 20134 Milano (IT)
(74) Representative: Torti, Carlo Maria Emilio
(86) International application number: PCT/IB2015/059077
(87) International publication number: WO 2016/083995

(56) References cited:
- WO-A1-2013/025904
- WO-A1-2014/140215
- US-A1- 2010 100 005
- US-A1- 2014 276 196

## Description

### Field of the invention

The present invention relates to a system and to the relative method of use for the dimensional measurement and clinical evaluation of allergic wheals generated by allergodiagnostic prick tests.

### Background art

The skin tests used for diagnostic purposes in the field of allergology are diversified by type and technique according to the clinical presentation to be evaluated and the potential causal element deemed responsible. Various techniques are employed: prick tests, patch tests (mainly used in the diagnostics of contact dermatitis and, although to a lesser extent, in that of food allergies and drug allergies), intradermal reactions (mainly used in the diagnostics of drug allergies and instead of prick tests when low power allergens are used) and scratch tests, which however are no longer used because of their low specificity.

In the scope of such tests, prick tests, which are considered as the basic skin test, are the ones most used in allergological diagnostics, above all in pediatrics.

The reasons for the widespread use of prick tests are mainly related to the following advantages that such tests offer:
- high efficiency or accuracy;
- simplicity of execution and interpretation;
- minimum invasiveness, to the extent of making them well acceptable in particular even to children;
- nearly no risk of side effects;
- low costs also for the material used (allergens, pricking and reading instruments).
Prick tests may induce an immediate skin reaction of mediated IgE type in sensitive subjects, thus determining the appearance of a wheal and of an erythema related to mast cell activation (there are approximately 5000-12000 per mm³ of mast cells in the skin, mainly located near vessels and nerve terminals). Indeed, the membrane IgE adherent to such cells which came into contact with the allergen being tested release vasoactive substances promoting both vasodilation and plasma extravasation.
Furthermore, prick tests may have other fields of application (e.g. in allergen standardization, in evaluating specific allergen reactivity during a specific immunotherapy course over time) as demonstration of its relevance in allergology and how good knowledge of the various aspects of such a technique is necessary. Histamine, the main released vasoactive substance, may determine, by means of axon reflexes, the release of P substance, which may in turn increase the effects of the immediate reaction and promote the release of further histamine by the mast cells.
Thus, both chemical and neurogenic mediators are involved in the scope of the immediate skin reaction induced by prick tests.
The immediate skin reaction may be followed by a tardive skin reaction, which may appear from 3 to 5 hours after the prick test was performed, with a maximum peak at the sixth-twelfth hour and with spontaneous resolution usually within 24 hours. Probably, the mast cells are the main cells at the base of the tardive reaction; indeed such a reaction appears nearly always after an immediate reaction in the genesis of which the mast cells play a primary role. These cells release chemotactic factors, cytokines and vasoactive substances which determine an increase of vascular leakage with consequent attraction of various cellular elements. Among these there are lymphocytes, eosinophils (with their secretion products, in particular the major basic protein and the cationic protein), neutrophils and macrophages and all these cells which would have also had an important role in the genesis of tardive type skin reaction.

The following definitions must be explained before turning our attention to the meaning of prick test sensitivity and specificity:
TP (true positive) = subject with positive prick test who is symptomatic (i.e. with symptoms triggered by the inhalant or food allergen which was positive to the prick test);
FP (false positive) = subject with positive prick test but who is asymptomatic (i.e. without symptoms triggered by the inhalant or food allergen which was positive to the prick test);
TN (true negative) = subject with negative prick test and who is asymptomatic;
FN (false negative) = subject with negative prick test but who is symptomatic;
PPV (positive predictive value) = probability that the subject is symptomatic because the prick test is positive;
NPV (negative predictive value) = probability that the subject is asymptomatic because the prick test is negative.

Thus, the sensitivity (Se) of the prick test is given by TP / (TP + FN) x 100 and the specificity (Sp) of the prick test is given by TN / (TN + FP) x 100.

In other words, if Se is the probability that a prick test is positive as the patient is symptomatic (the higher is Se, the lower are the number of false negatives) and Sp is the probability that a prick test is negative as the patient is asymptomatic (the higher is Sp, the lower are the number of false positives).

Ideally, a prick test should have 100% sensitivity and specificity for all allergens used. However, this is difficult to achieve in allergology because if the power of the diagnostic material is increased with the goal of increasing sensitivity, and consequently reducing FNs, its specificity may be decreased (thus increasing false positives). Therefore, the allergenic extract must have an optimal balance between sensitivity and specificity and good accuracy or efficiency so as to have the maximum number of TPs and the minimum number of FPs. Certainly, obtaining an optimal accuracy of a diagnostic material to be used for a prick test implies a biological standardization by means of validated methods by the manufacturers. In other words, the type of material used is important (if such a material is bad and poor in allergenic proteins the extract will not be representative of the allergenic proteins which it should contain) but so is the procedure used for making the final product which will subsequently be used to perform the prick test.

Probably, the use of recombining allergens as diagnostic material for prick tests, thus with a full knowledge of the allergens responsible for the appearance of clinical manifestations for each inhalant and food, is the end point of allergological diagnostics.

The studies from a high number of publications belong to this context. The article entitled "The predictive value of the skin prick test wheal size for the outcome of oral food challenges" by Verstege et al., Clin Exp Allergy. 2005 Sep;35(9):1220-6, emphasizes the importance of prick tests particularly in pediatrics in order to identify food allergies. The publication entitled "Clinical relevance is associated with allergen-specific wheal size in skin prick testing" by Haahtela et al., Clin Exp Allergy. 2014 Mar;44(3):407-16, further reasserts the preventive value of prick tests for evaluating allergic reactions given by the inhalation of allergens. The article entitled "The reproducibility of the allergy skin tests scoring and interpretation by board-certified/board-eligible allergists" by William A. et al., Ann Allergy Asthma Immunol 2002;89:368-371, highlights the difficulties related to the correct interpretation and reproducibility of prick tests for the various degrees of skin reaction.
Furthermore, the article entitled "The reproducibility of the allergy skin test scoring and interpretation by board-certified/board-eligible allergists", by W. McCann et al., ann Allergy Asthma Immunol 2002;89:368-371, clearly states the existence of a high number of variables involved in allergy skin tests and thus the absolute inaccuracy of the current methods for evaluating the respective results, which are strongly conditioned by the human factor.
From all these studies it results that prick tests have limits which are difficult to eliminate. In particular, it has been shown that in adult subjects, even if the prick test is performed by a capable operator using standardized technique, reproducibility with regards to the amount of inoculated allergen is poor. This shortcoming is difficult to eliminate because it depends on patient's individual features. A variability between subjects and, in the same subject, between one zone and the other of the skin of the forearm with regards to the amount of penetrated substance is thus present.

Prick tests are performed by putting a drop of allergenic extract on the anterior aspect of the forearm at a minimum distance of at least 3 cm between each drop. Afterward, the drop is pierced by means of a specific disposable sterile lancet (of the "Morrow-Brown" type in which the tip penetrates for 1 mm into the skin) perpendicularly with respect to the skin to then prick the skin itself; in a variant of this technique, the drop is pierced with an inclination of the lancet (in such a case, a different lancet with a wider tip is used, of the type of that used to draw blood from the child's fingertip in order to perform some types of tests) equal to 45 degrees and then the skin is raised (in both cases, drawing blood must be avoided). Subsequently, the drop is removed with 60 seconds from when the test is performed with gauze or cotton wool and the presence of erythema, itchiness and wheal is determined after 15 minutes.

Thus, in theory, any instrument capable of determining a small skin lesion could be used. In actual fact, it is apparent that this cannot be implemented because the amount of allergen which penetrates and thus also the type of skin response which is obtained, depends on the extension and the depth of the determined lesion. Furthermore, the degree of trauma determined on the skin by the instrument used determines a lesion which can also be source of incorrect interpretation in addition to being annoying and painful for the patient.
Moreover, results which are superimposable as a whole are obtained by analyzing the skin response to prick tests with histamine and that to prick tests with a control solution performed on the anterior aspect of the forearm using different pricking instruments although some of these instruments (in particular, Greer Track) determine responses which are different from the others. Clearly, this has practical implications of considerable importance because there could be different skin responses according to the different instrument used.
The above clearly shows the major limits, the difficulty in interpreting results and the poor reproducibility of this technique with the consequent error of diagnosis. Given the importance of prick tests it is thus necessary to find a system which can avoid the degree of approximation and uncertainty which still characterizes such a diagnostic method.
Examples of devices and methods for measuring allergic wheals are known from US 2010/0100005 A1.

### Summary of the invention

On the basis of the limits indicated above, it is the object of present invention to identify a system and a respective method of use for the correct identification and measurement and for the respective semi-automatic diagnosis of allergic wheals generated by prick tests.

It is a further object of the present invention to provide a system and a method which can be applied to all patients and to all particular skin types.

It is another object of the present invention to guarantee simplicity for the operator and reproducibility of the test method.

### Brief description of the figures

- Figure 1 shows a component of a device according to the present invention;
- figures 2 and 3 show a subject to whom a prick test was administrated;
- figures 4 and 5 show an enlargement of figures 2 and 3;
- figures 6, 7 and 8 show operating methods of a device according to the present invention;
- figure 9 shows a block diagram of operations performed by a device according to the present invention;
- figure 10 shows a side view of a device according to the present invention;
- figure 11 shows a perspective view of a device according to the present invention.

### Description of the invention

The present invention relates to a device for the dimensional determination and the clinical evaluation of allergic wheals caused by a prick test according to claim 1. Further disclosed are a test-strip and a method. The dependent claims refer to further advantageous aspects and preferred embodiments of the invention.

The following definitions and respective meanings are provided for a better understanding of the present invention:
**Wheal:** A skin reaction generated after a prick test. The size of the wheal, normalized with respect to the histamine wheal, is an indicator of the allergic patient's skin reactivity to the various allergens.
**Area of the wheal:** Skin portion on which an allergen was tested by means of a prick test.
**Allergens:** Substances of glycoprotein nature which can cause allergens.
**Test strip:** Adhesive strip or reference system which applied to the patient's forearm allows to correctly respect the distance for performing the prick tests.
**Skin Index:** An evaluation system which is based on the ratio between the area of the allergenic wheal with respect to that of the histamine wheal.

| | |
|---|---|
| **+** | **Wheal from ¼ to ½** |
| **++** | **Wheal from ½ to 1** |
| **+++** | **Wheal from 1 to 2** |
| **++++** | **Wheal greater than 2** |

**Targeting spot:** Geometric drawings (crosses, circle with crosses, etc.) for the necessary straightening of the image in order to regenerate the correct geometry of the wheal not deformed by the photographing perspective.

The area of the wheals is determined/supervised by the specialized physician. The device according to the present invention (also named "wheal meter" hereinafter) consists of:
- system for the acquisition of the images of the wheals;
- lighting system (flash and/or LED strings);
- high-definition digital photograph acquisition system (approximately 3264x2448 pixels) with "live view" functionality;
- supporting and positioning system of the acquisition sensor with respect to the patient's arm;
- test strip for positioning the test areas of the various allergens on the patient's arm.

The **test strip** includes:
**Cut-outs for allergens:** In an embodiment as disclosed, they may be arranged on two rows of six cut-outs characterized by a sequential numbering; six rows of two (total maximum number of 12 possible allergens to be tested per arm). Both the overall size of the test strip and that of the cut-outs may be determined as a function of the size of the part on which it must be applied, e.g. for adults or for children.
**Cut-outs for reference allergens:** In an embodiment as disclosed, they can be juxtaposed to form a row of two or more characterized by univocal graphic symbols (cross, asterisk etc.) for the positive control (histamine) and the negative control (glycerol).
**Targeting spots and reference color scale:** in an example embodiment as disclosed, they may be printed on the test strip and placed at the beginning of a side of the test strip; they may be represented by a square formed, in turn, by four squares of different color which are used to "calibrate" the color of the video camera before acquiring the image. On the other part, the test strip has a second square formed by two white squares and two black squares to "calibrate" the focusing of the video camera before acquiring the image, having the indication Dx for the right arm and Sn for the left arm.
**Allergen code:** The test strip may have a space adjacent to each window in which to write the identification code of the allergen.

The making of a test strip for adults and a test strip for children is contemplated. The device of the present invention also comprises a multimedia system provided with a software application for processing and determining the measurements of the wheals with the following functions:
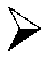 function of acquiring the image of the portion of the arm and of the test strip where the prick test was performed;
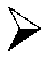 function of checking the image, by means of which the software checks the correct focusing, the sufficient light intensity and the absence of "blurred" effect on the acquired image;
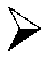 function of determining the geometry of the acquired image with which the software identifies the geometry of the test strip by searching for the targeting spots marked on it; the scale (size in pixel/mm) is calculated for each area in which the wheal appears;
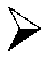 function of calibrating the color of the acquired image according to which the check and the color correction of the image are performed by detecting the reference colors shown on the test strip;
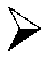 function of enhancing the discriminability of the wheal in order to allow the user to recognize the edges/perimeter of the wheal; the following operations are also performed in this step:
   (a)generating histograms;
   (b)separating the light frequency bands because in the processing of the three color bands (R, B, G) are separated for the quantitative analysis of each one;
   (c)calculating the optimal synthetic color palette for identifying the three objects (skin, erythema, wheal); the three previously obtained bands are "compared" with those of the reference wheals to define the characterization of the three classes;
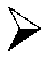 interactive graph functions for determining the absolute and relative measures of the single wheals resulting from the prick test.
   The software application further provides a series of functions for facilitating the drawing of the contour of the wheal and for visualizing the image, i.e. "zoom and pan" for varying the image, with which the application allows to enlarge the image of the wheal and to move it on the screen to aid the specialist in identifying the edge of the wheal and marking the points (circle and ellipsis) and/or tracing the drawing of the contour; and functions for adjusting contrast and brightness and drawing assistance functions, with which the operator can generate circles, ellipses or polygonal shapes useful to trace the contour of the identified wheal. According to the latter function, the operator "encircles" the wheal with a "stylus", with which the multimedia system is equipped, e.g. a tablet, identified for the first step. It is used as a pencil to draw on the screen, e.g. of the tablet;
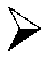 function of calculating the surface of the wheal allowed by the software, which contemplates the steps of measuring the surface of the wheal in mm, of normalized measuring with respect to the reference wheal obtained with histamine and converting to value according to the SKIN INDEX, and of calculating the ratio between the surface of the histamine reference and the one under examination. This allows to obtain a precise, accurate determination of the identified wheal and of the specific allergic reaction generated in the patient under examination.

Furthermore, the device according to the present invention also offers the possibility of managing the information related to the operator who performed the tests, the data acquired for each patient and the various prick tests to which the patient was subjected over time (Visit 1, 2, 3, ... as a diary). The data gathered for each visit/patient are managed by means of a database (db) to populate the historical archive. The data related to the prick tests and the respective images are stored for each patient in addition to personal data.

As indicated above, the present invention suggests a respective method for use in addition to a device for measuring the size of the allergic wheals. This will be illustrated in detail below with particular reference to the accompanying figures which are non-limiting and provided as example only.

The method as disclosed comprises the following main steps:
(A) acquiring the images of wheals and optimizing the optical parameters of said images;
(B) determining the geometry of the test strip based on the targeting spots provided on it and calculating the scale for each area in which the wheal appear;
(C) checking and correcting the image color by acquiring the reference colors shown on the test strip and distinguishing between wheal, skin and erythema;
(D) interactively measuring the shape and dimensions of the identified wheals by the operator.

The details related to the step (D) of interactive measuring are the crucial part of the method as disclosed.

### Operation

During the preliminary step, the operator must perform a series of operations to guarantee the good acquisition of the photographs and, by entering the data into the database, the correct correlation between the images and the customer's datasheet:
A first step contemplates accessing with credentials, during which the operator accesses the application with User ID and Password.

The patient's data are then entered.

The test strip is fixed to the patient's body part, preferably the arm used for the prick test. The test strip is preferably adhesive so as to allow its stable application and guarantee the correctness in the following image acquisition.

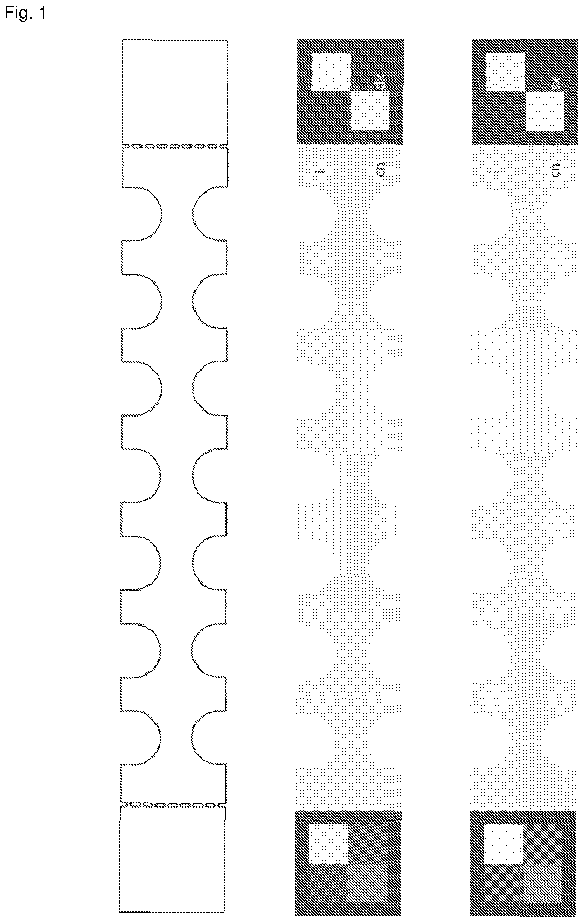

The step that follows contemplates associating the number of a given cut-out of the test strip to the administered allergen. A possible form of the test strip is shown in Fig. 1. The test strip shows sequential numbers at the holes. The operator, by means of the application, must associate the administrated allergen to the number of the corresponding cut-out.

At this point, after having applied the test strip, defined and taken note of the sequence of the allergens which will be tested, i.e. from 1 to 12 per arm (if required up to 24 tests can be performed at the same time by using the left arm, the right arm and test strip), the prick tests are performed in the specific spaces. The test strip may be punched in the joining points of the first colored square and of the second black and white square. So, when the prick tests are performed, the middle part of the test strip is detached and only the two squares at the two sides will remain. In this manner, the wheals can freely form and the image can be acquired without visual restriction by the test strip.

After the 15/20 minutes needed for the reaction to appear, the two squares which remain allow to align the "electronic grid/pointer" present in form of grid, for example green or blue, which appears on the screen, e.g. of the tablet, and to calibrate the apparatus before taking the photograph in terms of color and focus. At this point, the part of the patient's body used for the test is positioned for acquiring the image. The image to be acquired is shown on the screen with the superimposition of the green or blue grid which reproduces on the monitor, for example, the arm divided into the same areas which were defined by the test strip. This grid delimits the active acquisition field. The operator takes care of correctly positioning the forearm with respect to the references and checks correct focusing. By clicking on the specific button, the position of the forearm and the focusing will be accepted. Downstream of the confirmation command by the operator, the application will check position and focusing.

**Acquisition of the image:** The operator takes the photographs and immediately checks the acquired image also using the software tools indicated above; if the image is not satisfactory (movement of the patient, movement of the acquisition field, interruption of the lighting etc.) the operator can delete it and proceed with a new acquisition.

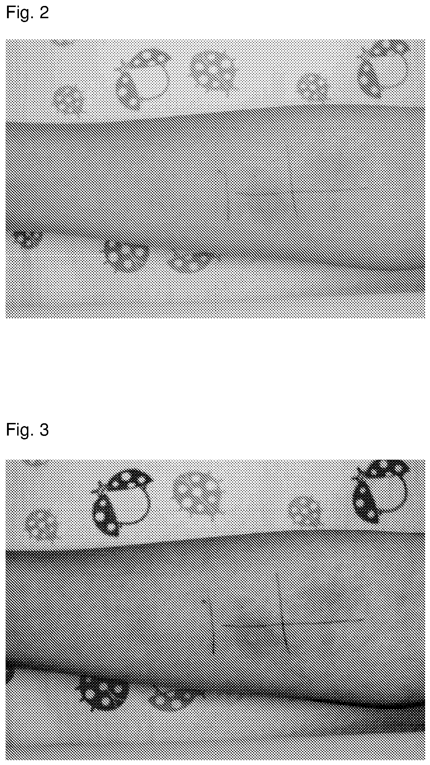

An example of initial image is shown in fig. 2, and the optimized image is shown in fig. 3.

**Acceptance of the image:** The operator must accept the acquired image, which will be saved and associated to the patient's datasheet.

**Drawing of the contours:** With the aid of the software tools described below, the operator can define the contours of the wheals.

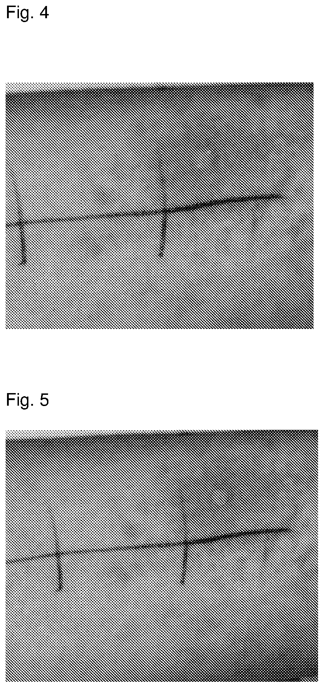

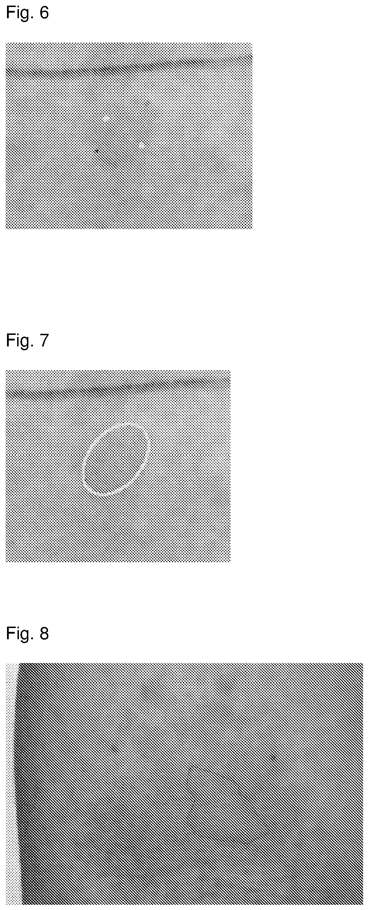

The first operation requested by the operator is to draw the contour of the reference wheals, which is a basic request for calculating the surfaces. The graphic interface offers some commands for facilitating the drawing:
**Circle:** The operator clicks on the two extremes of the diameter of the wheal (fig. 4), and the application generates the circle (fig. 5).
**Ellipsis:** The operator clicks on the extremes of the two axis (Fig. 6), and the application generates the ellipsis (Fig. 7).
**Polygonal:** The operator clicks on the points that determine the polygonal, and the application completes the drawing.
**Tracing:** the operator traces the contour of the wheal using a stylus by means of a series of points or a solid line (Fig. 8).

The operator must evaluate each drawing suggested by the application and accept it;
the operator proceeds by drawing the contour of the other wheals with the same methods described above;
if the operator does not detect the formation of the wheal or deems it not necessary to calculate, this choice must be indicated by clicking on the specific button; size 0 will be assigned to the examined wheal.

### Detailed operative example

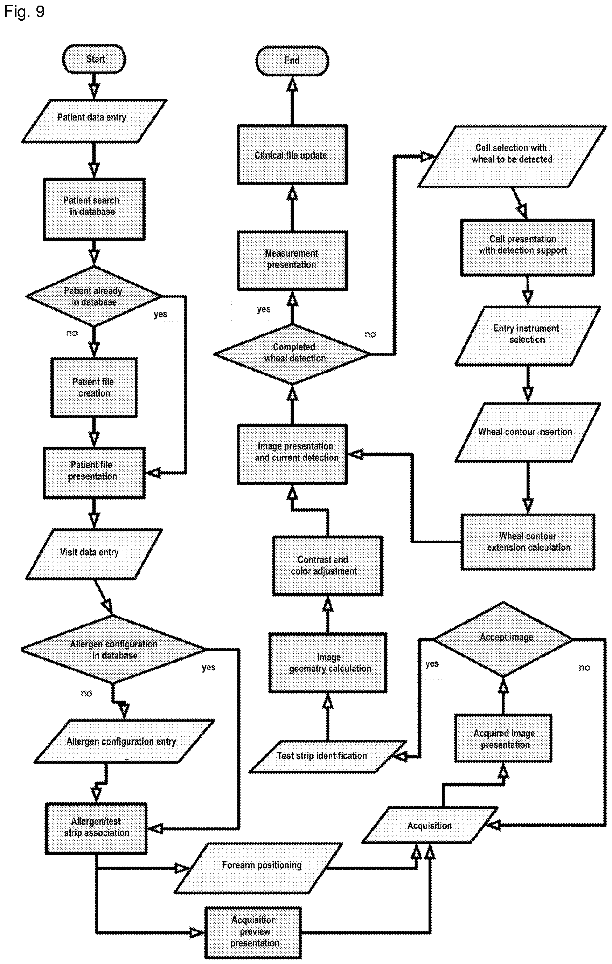

On the basis of that described above and for a better understanding of the interaction between hardware and software components of the device, reference will be made hereinafter to a detailed, non-limiting example of operation corresponding to the operations shown in the flow chart in fig. 9.

**Entering the patient's identification:** the user enters name or surname or identification number and the application suggests the patients compatible with the entered relevant data (e.g. relevant data for the patient, examination entities etc.) that have still to be specified (cf. comments in the project document).
- Loading patient data: if the patient is already in the database the patient's data are loaded.
- Entering patient data: if the patient is not already in the database the application supports the insertion of the relevant data.

**Entering examination data:** for each patient in the database, the application presents the possibility to:
- view the performed examinations: the application presents the list of examinations performed by the patient. Following the selection by the user of a particular examination of the list, the application presents the stored information concerning the selected examination;
- add a new examination: the application guides the user to create a new examination instance.
   ∘ The application pre-compiles the data field. The user may modify it.
   ∘ The application presents to the user various allergen configuration operations, identified by a code and by a description. The user may:
      ▪ select a configuration from the list. The configurations are stored in the database;
      ▪ create a new configuration: the user enters the allergens which will be inoculated in a table, the cells of which correspond to the inoculations. The allergens are selected from a list and stored in permanent manner in the database.

**Acquisition:** The application acquires an image of the concerned zone for the operation.

**Acquisition quality check:** The user may accept or reject the acquisition.

**Identification of the area of concern of the acquired image:** The application supports the identification, by the user, of the area of the image representing the surface under examination. The user identifies the four corners of the test strip by selecting them with the stylus on the touchscreen.

**Test strip detection check:** The application presents the test strip superimposed on the acquired image and asks for confirmation of the correct detection.

**Determination of the geometry of the image:** According to the coordinates of the test strip, the application estimates the perspective distortion introduced by the acquisition. Such an estimate is functional to the metric measurement.

**Automatic contrast correction:** The acquired image is processed in order to increase the discriminability of the wheal by the user.

**Wheal identification support:** The application provides the support to the user for entering the contours of the wheals in order to calculate the measurements thereof.

For each area identified on the test strip:
1. the user selects the region containing the wheal on the acquired image;
2. the application centers and re-sizes the region containing the wheal, so as to facilitate its identification;
3. the user identifies the contour of the wheal using the drawing identification functions offered by the application; the application supports the introduction of the contour of the wheal offering three instruments for selection:
   a. Circular wheal selection: the user identifies the contour of a circular shape wheal by selecting the diameter.
   b. Elliptic wheal selection: the user identifies the contour of an elliptic shape wheal by selecting the two axes.
   c. Polygonal wheal selection: the user identifies the contour of a polygonal shape wheal by selecting a closed broken line.
4. The application shows the selection and allows to accept it or reject it.

**Determining the measurements:** for each entered wheal the application provides the surface:
1. as absolute measure, in millimeters;
2. as normalized measurement and with respect to the reference wheals and to the Skin Index value.

**Permanence of the results:** the application saves the measurements of the wheals in the database.

**Backup:** the system makes periodical backups on the memory card of the hardware device and informs the user when the storage space is full.

**Exporting the results:** the application allows to generate a file containing the relevant information of a given examination.

The application allows to export files saving it on a path which can be accessed by the user.

**Printing:** a printing area of the results is contemplated and operative with the option of allowing the physician to sign.

**Area report:** an area is contemplated in which the activities of the device are evaluated (e.g.: number of patients included, statistics on the data to be defined, list).

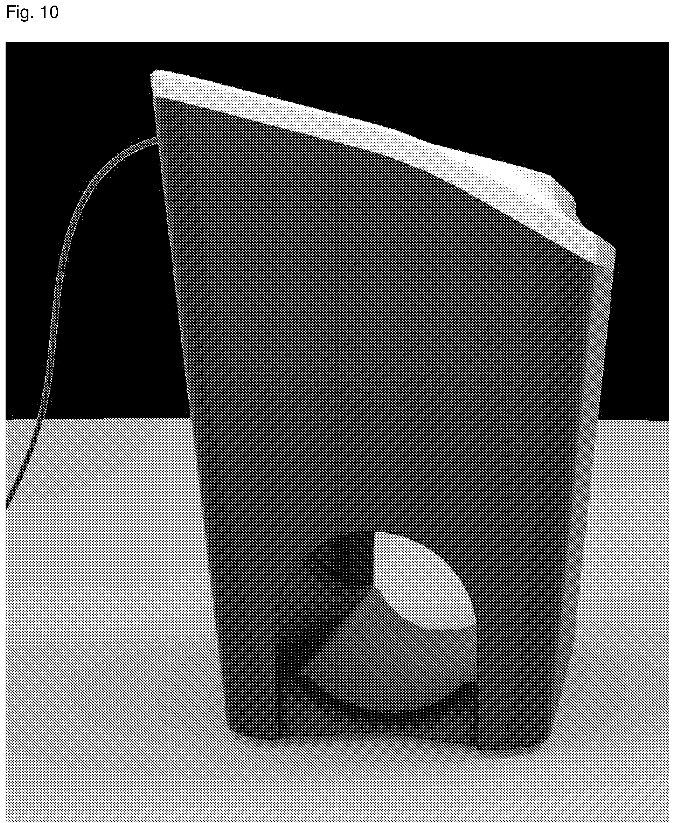

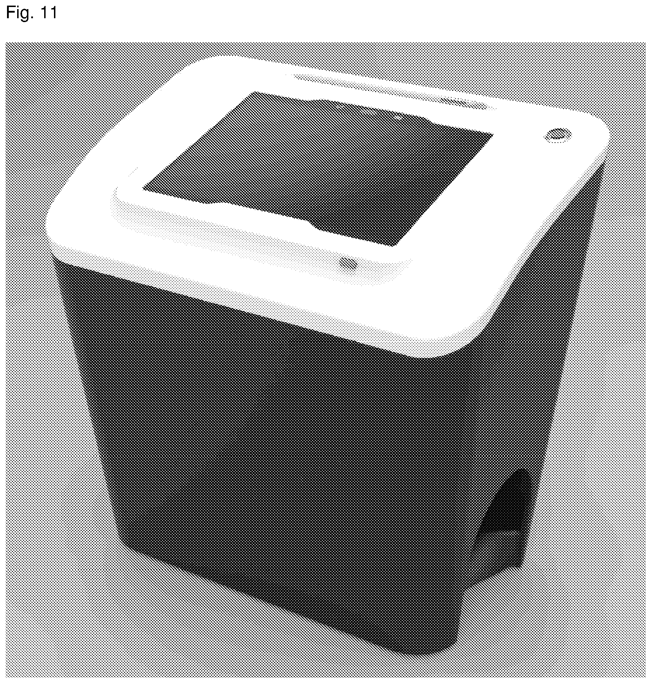

According to a preferred embodiment, the present invention contemplates a portable device for measuring the sizes of the wheals following prick tests performed on the patient's forearm. Such a device, comprising all the elements indicated above, is shown in figures 10 and 11.

As resulting from the description above, the device of the present invention and the method as disclosed thus offer, by means of the combination of image optimization and "normalization" of the same by using the test strip, a simple and accurate evaluation of the wheals resulting from prick tests, guaranteeing the exact location of such wheals, the precise distinction of the background and the rigorous determination of their size, thus ensuring a correct and reproducible semi-automatic diagnosis.

## Claims

1. Device for the dimensional determination and the clinical evaluation of allergic wheals caused by a prick test, comprising the following components:
(a) System for the acquisition of image of said wheals, said system comprising a video camera;
(b) A test strip for the positioning of the allergens on a part of a patient's body, preferably the forearm;
(c) A multimedia system comprising a software having the following functions:
- optimization of the acquired images
- identification of the wheals
- interactive measuring of the form and dimensions of the identified wheals
wherein said test strip comprises:
cut-outs used for
(i) administered allergens,
(ii) reference allergens,
**characterised in that** the test strip further comprises:
targeting spots and a reference-color scale,
said targeting spots and reference-color scale being provided for allowing to calibrate respectively the focusing and the color of the video camera before acquiring the image, said multimedia system comprising means for the calibration of the focus and color of the acquired image with control and correction of the image focus and color by means of the detection of respectively the targeting spots and reference colors on the test-strip thus allowing to distinguish each wheal from the skin and the erythema triggered by the administration of the allergen, obtaining a precise measure of the targeted area and allowing contemporarily an accurate clinical evaluation on the kind of allergic reaction.

2. Device according to claim 1,
**characterized in that** the test strip is fixable directly on the body part undergoing the prick test.

3. Device according to claim 1 or 2,
**characterized in that** the targeting spots and the reference-color scale are represented, on one end of the test strip, by a square composed by four sub-squares of different colors for adjusting the color setting of a camera acquiring the image.

4. Device according to claim 3,
**characterized in that** the other end of the strip is provided with a second square formed by 2 white and 2 black sub-squares, for adjusting the focus of the camera before the image acquisition.

5. Device according to one of claims 1 to 4,
**characterized in that** the test-strip further comprises an area adjacent to each cut-out for the identification code of each administered allergen.

6. Device according to one of claims 1 to 4,
**characterized in that** the test-strip is adhesive.

7. Device according to one of the preceding claims,
further comprising a system for the fixing and positioning of an image acquisition sensor with respect to the patient's body part.

8. Device according to one of the preceding claims 1 to 7,
**Characterized in that** the software comprises the following functions:
- Management of the operator and patient data;
- Determination of the geometry of the acquired image of a predefined wheal;
- Calculation the surface of the contoured wheal comprising normalized measuring with respect to a reference wheal and converting to a value according to a SKIN INDEX.

## Patentansprüche

1. Vorrichtung zur Abmessungsbestimmung und klinischen Bewertung allergischer Quaddeln, die durch einen Haut-Pricktest verursacht wurden, die folgende Bestandteile umfasst:
(a) System zum Erfassen eines Bilds der Quaddeln, wobei das System eine Videokamera umfasst;
(b) einen Teststreifen zum Positionieren der Allergene auf einem Teil des Körpers des Patienten, vorzugsweise dem Unterarm;
(c) ein Multimediasystem, umfassend eine Software mit den folgenden Funktionen:
Optimierung der erfassten Bilder
Identifikation der Quaddeln
interaktives Messen der Form und Abmessungen der identifizierten Quaddeln, wobei der Teststreifen Folgendes umfasst:
Ausschnitte, die für
(i) Verabreichte Allergenen,
(ii) Referenzallergenen verwendet werden,
**dadurch gekennzeichnet, dass** der Teststreifen ferner Folgendes umfasst:
Zielpunkte und eine Referenz-Farbskala,
wobei die Zielpunkte und die Referenz-Farbskala bereitgestellt sind, um ein Kalibrieren des Fokus bzw. der Farbe der Videokamera zu ermöglichen, bevor das Bild aufgenommen wird,
wobei das Multimediasystem Mittel zur Kalibrierung des Fokus und der Farbe des erfassten Bilds mittels Kontrolle und Korrektur des Bildfokus und der Farbe mittels der Detektion der Zielpunkte bzw. der Referenzfarben auf dem Teststreifen, wodurch ermöglicht wird, dass jede Quaddel von Haut und dem Erythem, das durch die Verabreichung des Allergens verursacht wurde, zu unterscheiden, wodurch eine präzise Messung des Zielbereichs erhalten wird und zeitnah eine präzise klinische Bewertung der Art der allergischen Reaktion ermöglicht.

2. Vorrichtung nach Anspruch 1,
**dadurch gekennzeichnet, dass** der Teststreifen direkt auf dem Körperteil, auf dem der Haut-Pricktest durchgeführt wird, befestigbar ist.

3. Vorrichtung nach Anspruch 1 oder 2,
**dadurch gekennzeichnet, dass** die Zielpunkte und die Referenz-Farbskala an einem Ende des Teststreifens durch ein Quadrat dargestellt sind, das aus vier Teilquadraten mit unterschiedlichen Farben zum Anpassen der Farbeinstellung einer Kamera besteht, die das Bild aufnimmt.

4. Vorrichtung nach Anspruch 3,
**dadurch gekennzeichnet, dass** das andere Ende des Streifens mit einem zweiten Quadrat zum Anpassen des Fokus der Kamera vor Aufnahme des Bilds versehen ist, das aus 2 weißen und 2 schwarzen Teilquadraten besteht.

5. Vorrichtung nach einem der Ansprüche 1 bis 4,
**dadurch gekennzeichnet, dass** der Teststreifen ferner einen Bereich neben jedem Ausschnitt für den Identifikationscode jedes verabreichten Allergens umfasst.

6. Vorrichtung nach einem der Ansprüche 1 bis 4, **dadurch gekennzeichnet, dass** der Teststreifen selbsthaftend ist.

7. Vorrichtung nach einem der vorstehenden Ansprüche, ferner umfassend ein System zum Befestigen und Positionieren eines Bilderfassungssensors in Bezug auf das Körperteil des Patienten.

8. Vorrichtung nach einem der vorstehenden Ansprüche 1 bis 7, **dadurch gekennzeichnet, dass** die Software die folgenden Funktionen umfasst:
- Verwaltung der Benutzer- und Patientendaten;
- Bestimmung der Geometrie des erfassten Bilds einer vordefinierten Quaddel;
- Berechnung der Oberfläche der konturierten Quaddel, umfassend die normalisierte Messung in Bezug auf eine Referenzquaddel und Umwandeln des Werts gemäß einem SKIN-INDEX.

## Revendications

1. Dispositif de détermination dimensionnelle et d'évaluation clinique des papules oedémateuses allergiques provoquées par un test de piqûre, comprenant les composants suivants :
(a) un système d'acquisition d'image des dites papules oedémateuses, ledit système comprenant une caméra vidéo ;
(b) une bandelette réactive pour positionner les allergènes sur une partie du corps d'un patient, de préférence sur l'avant-bras ;
(c) un système multimédia doté d'un logiciel avec les fonctions suivantes :
- optimisation des images acquises,
- identification des papules oedémateuses,
- mesure interactive de la forme et des dimensions des papules oedémateuses identifiées, ladite bandelette réactive comprend :
des découpes utilisées pour
(i) les allergènes administrés,
(ii) des allergènes de référence,
**caractérisé en ce que** la bandelette réactive comprend en outre :
des points de ciblage et une échelle de couleur de référence,
lesdits points de ciblage et l'échelle de couleur de référence étant prévus pour permettre de calibrer respectivement la mise au point et la couleur de la caméra vidéo avant l'acquisition de l'image, ledit système multimédia comprenant des moyens pour calibrer la mise au point et la couleur de l'image acquise avec contrôle et correction de la focalisation de l'image et de la couleur au moyen de la détection respectivement des points de ciblage et des couleurs de référence sur la bandelette réactive, permettant ainsi de distinguer chaque papule oedémateuse de la peau et de l'érythème provoqué par l'administration de l'allergène, en obtenant une mesure précise de la zone ciblée et permettant simultanément une évaluation clinique précise du type de réaction allergique.

2. Dispositif selon la revendication 1,
**caractérisé en ce que** la bandelette réactive peut être fixée directement sur la partie du corps soumise au test de piqûre.

3. Dispositif selon la revendication 1 ou 2,
**caractérisé en ce que** les points de ciblage et l'échelle de couleur de référence sont représentés, à une extrémité de la bandelette réactive, par un carré composé de quatre sous-carrés de couleurs différentes pour ajuster le réglage des couleurs d'une caméra acquérant l'image.

4. Dispositif selon la revendication 3,
**caractérisé en ce que** l'autre extrémité de la bandelette est munie d'un second carré formé de 2 sous-carrés blancs et de 2 sous-carrés noirs, pour ajuster la mise au point de la caméra avant l'acquisition de l'image.

5. Dispositif selon l'une quelconque des revendications 1 à 4,
**caractérisé en ce que** la bandelette réactive comprend en outre une zone adjacente à chaque découpe pour le code d'identification de chaque allergène administré.

6. Dispositif selon l'une quelconque des revendications 1 à 4,
**caractérisé en ce que** la bandelette réactive est adhésive.

7. Dispositif selon l'une quelconque des revendications précédentes,
comprenant en outre un système pour la fixation et le positionnement d'un capteur d'acquisition d'images par rapport à la partie du corps du patient.

8. Dispositif selon l'une des revendications précédentes 1 à 7,
**caractérisé en ce que** le logiciel comprend les fonctions suivantes :
- la gestion des données de l'opérateur et du patient ;
- la détermination de la géométrie de l'image acquise d'une papule oedémateuse prédéfinie ;
- le calcul de la surface de la papule oedémateuse contournée comprenant la mesure normalisée par rapport à une papule oedémateuse de référence et la conversion en valeur en fonction de l'INDICE DE PEAU.
